**Europäisches Patentamt**

**European Patent Office**

**Office européen des brevets**

(11) Publication number: **0 165 751**
**A2**

(12) **EUROPEAN PATENT APPLICATION**

(21) Application number: **85304078.0**

(22) Date of filing: **10.06.85**

(51) Int. Cl.⁴: **A 61 M 1/14**
**A 61 M 1/34**

(30) Priority: **18.06.84 CS 4618/84**

(43) Date of publication of application:
**27.12.85 Bulletin 85/52**

(84) Designated Contracting States:
**DE FR GB NL SE**

(71) Applicant: **CHIRANA Vyzkumny ustav zdravotnicke
techniky koncernova ucelova** organizace
**No 3 Kamenice**
Brno(CS)

(72) Inventor: **Skala, Miroslav**
**No. 28 Dedicka**
**Brno(CS)**

(74) Representative: **Griffin, Kenneth David** et al,
**Saunders & Dolleymore 2 Norfolk Road
Rickmansworth Hertfordshire WD3 1JH(GB)**

(54) **Apparatus for the purification of blood.**

(57) The apparatus comprises a monitor (1) which consists of at least one replaceable basic unit (2) communicating with a hydroblock (3). The number of the basic units is chosen partly according to the kind of dialysation programme, partly according to the number of the quantities to be monitored and partly according to the volume of the pumped medium; one basic unit weighs less than 7 kg. Dialysation path is only outside the apparatus in a hydroblock module which enables easy and fast sterilization.

FIG 2

APPARATUS FOR THE PURIFICATION OF BLOOD

The invention relates to an apparatus for the purification of blood by haemodialysis, haemofiltration, haemoperfusion and also plasmapheresis, the apparatus comprising a monitor and a water treatment system upstream of the monitor.

Dialysis treatment and the apparatus therefor are very expensive. The function of a live human kidney can at least partly be replaced either by diffusion (haemodialysis) or by filtration (haemofiltration) or by the separation of substances from blood through activated carbon or resins (haemoperfusion), or by perspectively fractioned separation of individual blood components a process of blood detoxication which has already been used earlier, either by centrifugation or by membrane separation (plasmapheresis). The basic function of kidneys is glomerular filtration. If, due to a disease, the number of glomerules and thus the glomerular filtration drop to one quarter of the original values, only one quarter of sodium will be filtrated in the glomerules. Nephron, the functional unit of a kidney, controls the extraction of potassium and acids with the same perfection. One of the main waste products in protein metabolism is the urea. High blood urea results in clinical symptoms the sum of which is called uraemia. The concentration of waste products of protein metabolism in the blood increases, especially the concentration of urea, creatinine, phosphorous and potassium; also the concentration of sodium and possible acidity of the inner medium, i.e. the concentration of hydrogen ions vary. Thus kidney failure is a condition when the kidneys are not able to maintain constant composition of the inner medium even under basic conditions of life, i.e. when a person is healthy, is relatively physically at rest, and does not eat more than 0.5 g of proteins per 1 kg of his or her weight, i.e. the so-called biological minimum. Insufficiency of kidneys can be treated by a conservative method or by a method by which the blood is artificially purified as a substitution for the function of a natural kidney (peritoneal dialysis, haemodialysis, haemofiltration, haemoperfusion) or by transplantation of living kidneys. Artificial kidney is an apparatus which performs the function of a natural kidney. It comprises a dialyser proper, i.e. a part for the dialysis proper, transferring waste matter through a semipermeable membrane, and an apparatus called monitor preparing automatically the dialysation solution from water and salt concentrates protecting the organism of the treated person against damage. Dialysis is defined as a process in which a semipermeable membrane separates a solution of colloidal matter from a solution of crystalloidal matter. The physical principle of dialysis is a process called diffusion. Natural kidney, however, operates on the principle of filtration. Artificial simulation of the glomerular filtration of a living kidney by an artificial kidney was not possible in the early days because of low ultrafiltration capability of the used membranes. This was made possible after development of membranes enabling a higher hydrodynamic rate of flow for water and with a substantially higher permeability had been completed. If by haemofiltration the same quantity of waste matter is to be separated during the same time like during haemodialysis, 20 litres of water and blood must be filtrated and as much water and salts must be continuously delivered as is removed. Haemoperfusion is a treatment method in which endogenic and exogenic poisons are removed from blood by direct contact of blood with a sorbent. The blood whose coagulability is reduced by the addition of heparin (anticoagulant) is led out of the organism into extracorporeal circulation, in which it passes through a vessel (cartridge), containing particles of a sorbent, and returns back into the organism circulation. For haemoperfusion the apparatus called artificial kidney can be used provided with a blood pump, a blood-pressure meter, a detector of air bubbles in blood ways and a pump for heparin. Haemoperfusion is an effective method for the treatment of liver and kidney failure.

Plasmapheresis has been used already many years ago to remove toxic matters or harmful matters present in plasma.

Known haemodialysis apparatusses – monitors are composed of the following parts: flow indicator, conductivity measuring assembly of dialysation solution, dialysation solution temperature measuring device, dialysation solution pressure measuring instrument, haemoglobin detector, air bubble leakage detector in the blood ways, blood pressure meter for extracorporeal circulation, blood pressure meter on the arterial side of the extracorporeal circulation. etc. They are designed as universal apparatusses placed in large cabinets and as a rule they are provided with the latest microelectronics, th hydromechanical part being arranged inside the apparatus and designed for all the patients treated by means of this apparatus.

-4-

Infection by hepatitis B is prevented usually by sterilization and disinfection solution. Such solutions are known where a part of the dialysation way is replaceable and a part of this way is used for all the patients. The possibility of infection is substantially decreased here but not excluded. All these apparatusses - monitors, respecting the programs of the dialysis, haemofiltration, haemoperfusion, are independent apparatusses, dialysation modulus and blood modulus are designed as two blocks which are different due to their connection.

The above-mentioned shortcomings are avoided by the apparatus according to the present invention comprising a monitor consisting of at least one replaceable basic unit composed of a body to the upper front part of which is connected a high flow rate pump for blood or dialysate, to the front part of which are connected a low flow rate pump for infusion, for controlled ultra-filtration or for bicarbonate, a pressure sensor for blood or dialysate, a socket for hydroblock and a throttle. To the upper rear part of the body there is connected indication and parameters selection block. In the inner part of the body is installed the block of action electronics.

A further feature of this invention is that the hydroblock is formed by at least one cassette. Another feature of the present invention is that the conductivity cassette contains a water inlet hydraulically connected to the concentrate inlet at the point following the first jet of the concentrate inlet and further on connected through the first conductivity sensor with inlet into the first vessel in its upper part. The lower part of the first vessel is provided with the firt vessel outlet that is hydraulically connected to the high flow rate pump inlet, with whose outlet there is hyraulically connected the inlet in the upper part of the second vessel from where also the first outlet to the pressure sensor is led. The bottom of the second vessel is provided with the second vessel outlet which through the second conductivity sensor is hydraulically connected both to the by-pass outlet and to the first dialyser outlet. The upper part of the second vessel is provided with a deaeration outlet while the outlet of the first conductivity sensor and the outlet of the second conductivity sensor are electrically connected to the first connector.

A feature of the present invention is also that the underpressure cassette contains a dialyser inlet hydraulically connected with the second dialyser outlet, by-pass inlet, the third dialyser outlet and deaeration inlet are hydraulically connected, each separately, into the upper part of the third vessel provided with the second outlet for the pressure sensor. The bottom of the third vessel is provided with an outlet which is hydraulically connected through the blood detector to the second inlet of the high flow rate pump while the blood detector outlet is electrically connected to the second connector. Further on according to the present invention a feature of the apparatus is that the blood cassette includes the first level detector and the second level detector which are fixed in the holder of removable venose sac whose upper part is provided with infusion outlet, second outlet and first outlet which is hydraulically connected with the third outlet to the pressure sensor. The third outlet from the venose sac is inserted into the

-6-

bubble detector, the outlet of the first level detector, the outlet of the second level detector and the outlet of the bubble detector are electrically connected to the third connector.

A further feature of the apparatus according to the present invention is that the hydroblock for haemodialysis realised by the blood cassette, underpressure cassette, conductivity cassette and hydraulic interconnections so that the conductivity cassette by-pass outlet is hydraulically interconnected by by-pass interconnection with the underpressure cassette by-pass inlet, the first dialyser outlet of the conductivity cassette is hydraulically connected by dialyser interconnection with the dialyser inlet of the underpressure cassette and the deaeration outlet of the conductivity cassette is hydraulically connected through deaeration interconnection containing the second jet with underpressure cassette deaeration inlet.

Another feature of the apparatus according to the present invention is that the monitor is set-up of three basic units upon which the hydroblock for haemodialysis is slid so that into the socket for hydroblock of the first basic unit there is slid the first connector of the conductivity cassette, into the socket for hydroblock of the second basic unit there is slid the second connector of the underpressure cassette, into the socket for hydroblock of the third basic unit there is slid the third connector of the blood cassette and the first outlet of the conductivity cassette is connected to the pressure sensor of the first basic unit, the second outlet of the underpressure cassette is connected to the pressure sensor of the second basic unit, the third outlet of the blood cassette is connected to the pressure sensor of the third basic unit. Inlet of the concentrate of the conductivity cassette is hydraulically connected through the throttle valve of the first basic unit to the container with the dialysate. The first inlet and the first outlet of the high flow rate pump are connected to the high flow rate pump of the first basic unit. By-pass interconnection and dialyser interconnection are led through the throttle valve of the second basic unit. To the second dialyser output and to the third dialyser output of the underpressure cassette there is connected the dialysation part of the dialyser, the second inlet of the high flow rate pump is connected to the high flow rate pump of the second basic unit, the second outlet of the high flow rate pump brought to the drain for the dialysate. The third outlet of the venose sac of the blood cassette is connected through the throttle valve of the third basic unit to the venose inlet, the second outlet of the venose sac of the blood cassette is connected through dialyser blood part and high flow rate pump of the third basic unit to the arterial inlet.

Monitor includes an assembly of several quite identical basic units which are arbitrarily interchangeable and weight of which is less than 7 kg. In case of a failure the defective basic unit may thus be replaced by the operator while the substitute unit also serves as a service gauge.

Hydromechanical parts, due to their location out of monitor, enable easy repairs and inspection, their cassette design and various combination of hydraulic interconnections enable the apparatus to be used both for all ordinary dialysation programmes but it can also be used for other purposes, e.g. for blood pumping or for diluting and mixing liquids. The design of the hydroblock and small dimensions of its parts make it possible to shorten idling of the apparatus and thus to exploit it for a longer time so that after passing the active programme it is possible to change the cassettes and the interconnections for new ones - sterilized ones, or other ones which are needed for the concrete patient. Sterilisation time will become shorter even with conventional sterilisation. It is possible to make in such way up to four dialyses.

The design of the blood cassette makes it possible to decrease substantially the blood volume in sets by up to 1/3 and to operate practically with any kind of venose sac.

The design of the apparatus makes it possible to set the critical values of individual parameters outside of the apparatus by the control elements or also by the connected processor control system and also monitoring of several parameters at the same time - according to the number of associated basic units.

The design of the monitor further makes possible the monitoring of the pressure before dialyser, double monitoring the conductivity, multiple monitoring of the blood level which secures high safety of the patient.

The invention will now be described by way of example, with reference to the accompanying diagrammatic drawings, in which:

Figure 1 is an axonometric view of the dialyser monitor set-up of three basic units and hydroblock;

Figure 2 is an axonometric view of the basic unit;

Figure 3 shows the hydromechanical connection of the hydroblock for haemodialysis and mutual interconnection and the cassettes are separated by a dashed line; and

Figure 4 is an axanometric view of monocassette performance, i.e. assembly of the hydroblock for haemodialysis into one total and shows its sliding of three basic units designed for this performance.

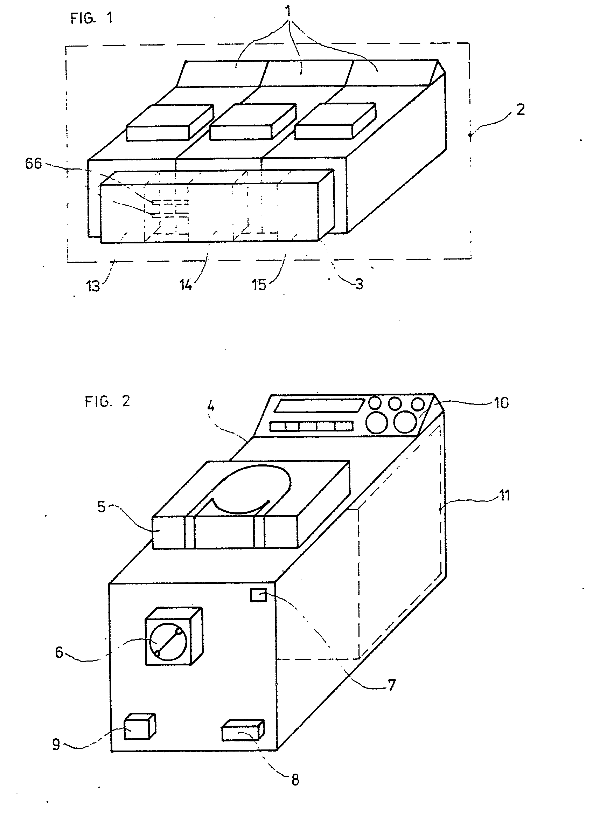

Monitor 2 (Fig. 1) connected for haemodialysis is composed of three identical basic units 1 to the faces of which is connected a hydroblock 3 composed of a conductivity cassette 13, an underpressure cassette 14, a blood cassette 15 and hydraulic connections 66.

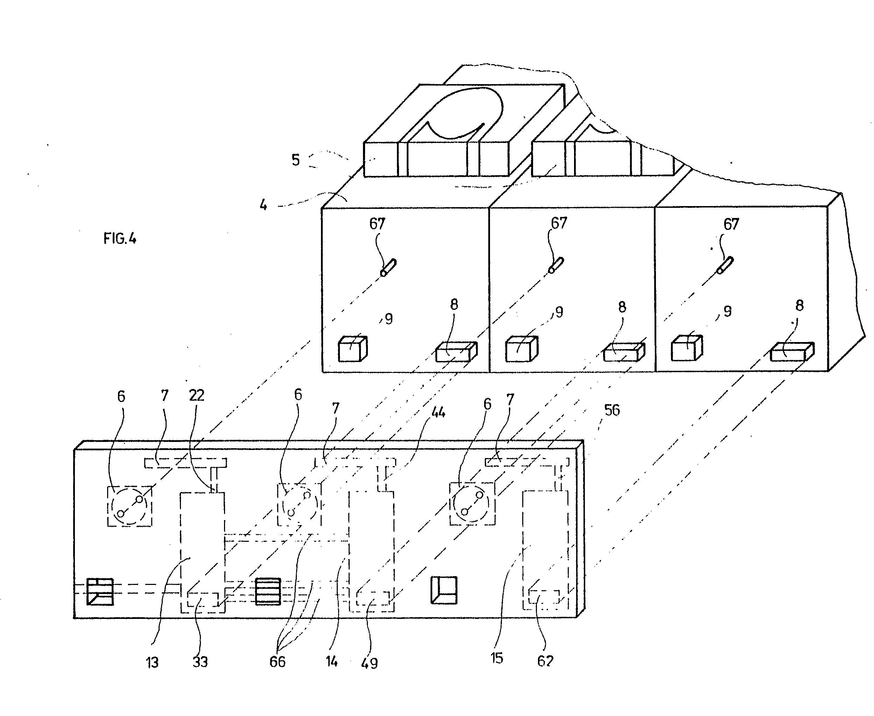

Each basic unit 1 (Fig. 2) weighs less than 7Kg and is composed of a body 4 to whose upper front part is connected a high flow rate pump 5. To the face of the body 4 is connected a low flow rate pump 6 driven by a shaft 67 (Fig. 4), a pressure sensor 7, a socket 8 for the hydroblock 3 and a throttle valve 9. To the upper rear part of the body 4 is connected a block 10 for indication and choice of parameters. In the inner part of the body 4 is installed an action electronics block 11.

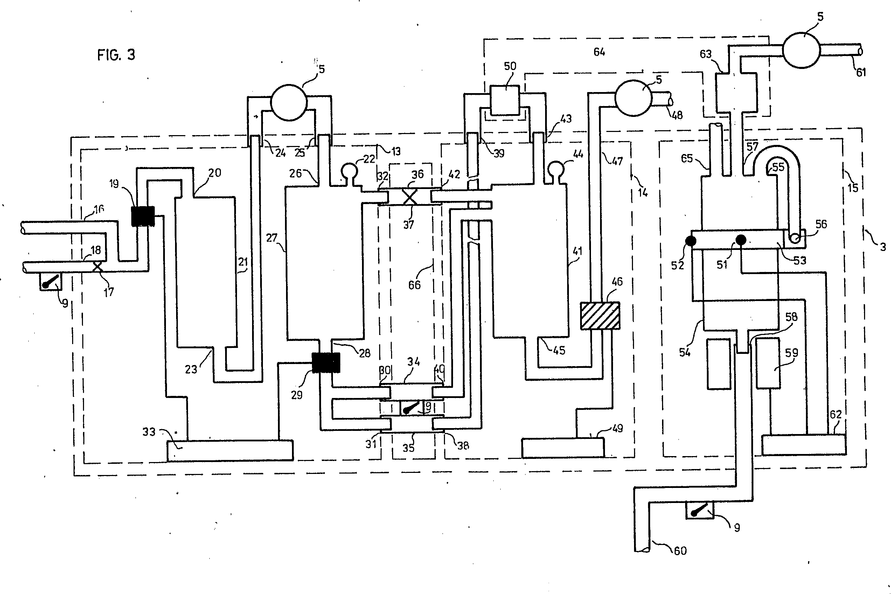

The conductivity cassette 13 (Fig. 3) contains a water inlet 16 which is hydraulically connected to a concentrate inlet 18 at a point downstream of a first nozzle 17 of the concentrate inlet 18. These inlets are further connected through a first conductivity probe 19 with an inlet 20 into a first vessel 21 in its upper part. The lower part of the first vessel 21 is provided with an outlet 23 which is hydraulically connected to a first inlet 24 of the high flow rate pump 5 to whose first outlet 25 is hydraulically connected an inlet 26 in the upper part of a second vessel 27 from where is also led the first outlet 22 to the pressure sensor 7. The bottom of the second vessel 27 is provided with an outlet 28 which through a second conductivity probe 29 is hydraulically connected to a by-pass outlet 30 and also to a first dialysate outlet 31. In the upper part of the second vessel 27 is a deaeration outlet 32 while signal outputs of the first and second conductivity probes 19 and 29 are electrically connected to a first connector 33.

The underpressure cassette 14 contains a dialysate inlet 38 hydraulically interconnected with a second dialysate outlet 39. A by-pass inlet 40, a third dialysate outlet 43 and an deaeration inlet 42 are hydraulically connected, each separately, to the upper part of a third vessel 41 which is provided with a second outlet 45, which is hydraulically connected through a blood detector 46 to a second inlet 47 of the high flow rate pump 5. The signal output of the blood detector 46 is electrically connected to a second connector 49.

The blood cassette 15 contains a first level detector 51 and a second level detector 52 which are fixed in a holder 53 for a removable venose sac 54. In the upper part of the venose sac 54 is an infusion inlet 65, a second outlet 57 and a first outlet 55 that is hydraulically connected with a third outlet 56 to the pressure sensor 7. A third outlet 58 from the venose sac 54 is inserted into a bubble detector 59. The signal outputs of the first level detector 51, of the second level detector 52 and of the bubble detector 59 are electrically connected to a third connector 62.

The hydroblock 3 in connection for haemodialysis in which the hydraulic interconnections 66 between the cassettes are arranged so that the by-pass outlet 30 of the conductivity cassette 13 is hydraulically connected through a by-pass interconnection 34 with the by-pass inlet 40 of the underpressure cassette 14. The first dialysate outlet 31 of the conductivity cassette 13 is hydraulically connected through a dialysate interconnection 35 with the dialysate inlet 38 of the underpressure cassette 14 and the deaeration outlet 32 of the conductivity cassette 13 is hydraulically connected through a deaeration interconnection 36 containing a second nozzle 37 with the deaeration inlet 42 of the underpressure cassette 14.

The hydroblock 3 for haemodialysis (Figure 4) is connected to the faces of the basic units 1 such that into the socket 8 of the first basic unit 1 is inserted the first connector 33 of the conductivity cassette 13, into the socket 8 of the second basic unit 1 is inserted the second connector 49 of the underpressure cassette 14, into the socket 8 of the third basic unit 1 is inserted the third connector 62 of the blood cassette 15. The first outlet 22 of the conductivity cassette 13 is connected to the pressure sensor 7 of the first basic unit 1, the second outlet 44 of the underpressure cassette 14 is connected to the pressure sensor 7 of the second basic unit 1, the third outlet 56 of the blood cassette 15 is connected to the pressure sensor 7 of the third basic unit 1. The concentrate inlet 18 (Figure 3) of the conductivity cssette 13 is hydraulically connected through the throttle valve 9 of the first basic unit 1 to the tank with dialysate. The first inlet 24 and the first outlet 25 of the high flow rate pump 5 are connected to the high flow rate pump 5 of the first basic unit 1. To the second dialysate outlet 39 and the third dialyser outlet 43 of the underpressure cassette 14 is connected the dialysation part 50 of the dialyser 64. The second inlet 47 of the high flow rate pump 5 is connected to the high flow rate pump 5 of the second basic unit 1 through whose throttle 9 are led the by-pass interconnection 34 and the dialysate interconnection 35, the second outlet 48 of the high flow rate pump 5 is brought to the dialysate drain. The third outlet 58 of the venose sac 54 of the blood cassette 15 communicates with a venose inlet 60 which is controlled by the throttling valve 9 of the third basic unit and leads to the patient. The second outlet 57 of the venose sac 54 of the blood cassette 15 is led through the blood part 63 of the dialyser 64 and the high flow rate pump 5 of the third basic unit 1 to the arterial inlet 61.

Heated-up and deaerated water is brought into the monitor through the water feed 16. Due to the underpressure formed in the hydroblock system by the high flow rate pump 5 of the first basic unit 1 the concentrate is fed into the hydroblock through the throttle valve 9, concentrate inlet 18 and the first nozzle 17. The dialysation solution formed in this way flows through the inlet 20 into the first vessel 21. In the inlet 20 is measured the conductivity of the unmixed dialysation solution by means of the conductivity probe 19. From the data given by this probe the throttle valve position 9 is derived at through the electric connection of the first conductivity probe 9 with the first connector 33 which enables its interconnection with the block 11 of the action electronics.

The dialysate further proceeds through the first vessel 21 and its outlet 23 into the first inlet 24 of the high flow rate pump 5 by which the dialysate is pumped through its first outlet 25 and through the inlet 26 into the second vessel 27. In the second vessel 27 through the first outlet 22 to the pressure sensor 7 is measured the pressure of the dialysate upstream of the dialyser. From the second vessel 27 is at the same time removed surplus air which flows through the deaeration outlet 32, through the deaeration interconnection 36 provided with the second nozzle 37 and through the deaeration inlet 42 into the third vessel 41.

Dialysate from the second vessel 27 leaves through the outlet 28 through the second conductivity probe 29 which, when the throttle valve is in its first position, controls the position of the throttle valve 9 in the second basic unit 1, in such a manner that the dialysate may flow through the by-pass outlet 30 the by-pass interconnection 34 and by-pass inlet 40 into the third vessel 41.

When the throttle valve 9 is in its second position the dialysate leaves through the first dialyser outlet 31, dialyser interconnection 35 and dialysate inlet 38 into the second dialysate outlet 39 while it further passes through the dialysation part 50 of the dialyser 64 to the third dialysate outlet 43 and from there into the third vessel 41. In the third vessel 41 is measured the pressure of the dialysate behind the dialyser 64 through the second outlet 44 on the pressure sensor 7 in the second basic unit 1.

The used dialysate is further fed through the outlet 45 from the third vessel 41, through the blood detector 46, whose signal is evaluated in the block 11 of the action electronics, into the second inlet 47 of the high flow rate pump 5 in the second basic unit 1 and from there through the second outlet 48 of the high flow rate pump 5 into the waste channel.

The flow of blood in the blood cassette 15 is as follows: Blood is sucked through the arterial inlet 61 by the high flow rate pump 5 of the third basic unit 1 into the blood part of the dialyser 64 from where it flows into the second outlet 57 of the venose sac 54. The removable venose sac 54 is provided with an infusion outlet 65 that serves for infusion. Further by means of the first outlet 55 of the venose sac 54 it is possible to measure the pressure in the removable venose sac which is realized through the third outlet to the pressure sensor 7 in the third basic unit 1. The removable venose sac 54 is held by the holder 53 which is provided with the first level detector 51 and the second level detector 52. Both detectors serve to evaluate the blood level by means of two independent principles. Their electric signals are conducted by means of a third connector 62 to further processing. The blood that passed through the cleaning process flows further from the removable venose sac 54 into the third outlet 58 of the venose sac 54 and through the bubble detector 59 (which is connected with the block 11 of the action electronics through the third connector 62) and flows through the throttling valve 9 in the third basic unit 1 to the venose inlet 60.

For haemoperfusion the apparatus is composed of one or two basic units upon which the blood cassettes are slid. For haemofiltration is used a system of two or more basic units and all kinds of cassettes: blood cassette, underpressure cassette and conductivity cassette. The number of basic units is determined in this case by the amount of the passing medium. For emergency use, supposing that the number of the monitored quantities is reduced, two basic units will suffice. For plasmapheresis at least four basic units are used.

With any arrangements the apparatus may be used for extracorporeal blood pumping, e.g. in cardiosurgical operations, for which purpose one basic unit is sufficient. In case of necessity the basic unit may be used in emergency cases, e.g. in cases of medicine poisoning, when time plays a decisive role.

The unit may be used, e.g. in fast medical help vehicles, with battery power supply. The apparatus will find wide application for diluting and mixing aggressive liquids especially in chemical and food industry.

CLAIMS

1. Apparatus for the purification of blood designed to used for haemodialysis, haemofiltration, haemoperfusion, and also plasmapheresis comprising a monitor and a water treatment plant situated upstream thereof, characterised in that the monitor (2) comprises at least one replaceable basic unit (1) communicating with a hydroblock (3).

2. Apparatus according to Claim 1, characterised in that the basic unit is composed of a body (4) to the upper front part of which is connected a high flow rate pump (5) for blood or dialysate to the face of the body (4) are connected a low flow rate pump (6) for infusion, controlled ultrafiltration or bicarbonate, a pressure sensor (7) for blood or dialysate, a socket (8) for the hydroblock (3) and a throttle valve (9), and to the upper rear part of the body (4) is connected a block (10) for indication and selection of parameters, while in the inner part of the body (4) is installed a block (11) of operational electronics.

3. Apparatus according to Claim 1, characterised in that the basic unit (1) comprises a body (4) in the upper front part of which is fixed a high flow rate pump (5) for blood or dialysate, to the body (4) face is fixed the shaft (67) of a low flow rate pump (6) for infusion controlled ultrafiltration or bicarbonate, a socket (8) for the hydroblock (3) and a throttle valve (9), while to the upper rear part of the body (4) is fixed a block (10) for indication and selection of parameters, and in the inner part of the body (4) is installed a block (11) of operational electronics.

4. Apparatus according to Claim 1, characterised in that the hydroblock (3) is formed by at least one cassette which serves for sensing the set quantities, such as pressure, temperature and conductivity, and for hydromechanical distribution of the medium.

5. Apparatus according to Claim 4, characterised in that it comprises a conductivity cassette (13) which contains a water inlet (16) which is hydraulically connected to a concentrate inlet (18) at a point downstream of a first jet (17) of the concentrate inlet (18) which is further connected through a first conductivity probe (19) with an inlet (20) into a first vessel (21) in its upper part, the lower part of the first vessel (21) is provided with an outlet (23) which is hydraulically connected with a first inlet (24) of a high flow rate pump (5) with whose first outlet (25) is hydraulically connected an inlet (26) in the upper part of a second vessel (27) in which part is also situated a first outlet (22) to a pressure sensor (7), the bottom of the second vessel (27) is provided with an outlet (28) which through a second conductivity probe (29) is hydraulically connected to a by-pass outlet (30) and to a first dialysate outlet (31), in the upper part of the second vessel (27) is situated a deaeration outlet (32), while signal outputs of the first conductivity probe (19) and the second conductivity probe (29) are electrically connected to a first connector (33).

6. Apparatus according to Claim 4, characterised in that it comprises an underpressure cassette (14) which contains a dialysate inlet (38) hydraulically connected to a second dialysate outlet (39), a by-pass inlet (40), a third dialysate outlet (43) and a deaeration inlet (42) are, each separately, hydraulically connected to the upper

-18-

part of a third vessel (41) provided with a second outlet (44) to a pressure sensor (7), in the bottom of the third vessel (41) is situated an outlet (45) that is hydraulically connected through a blood detector (46) to the second inlet (47) of the high flow rate pump (5), while the output of the blood detector (46) is electrically connected to a second connector (49).

7.    Apparatus according to Claim 4, characterised in that it comprises a blood cassette (15) which contains a first level detector (51) and a second level detector (52) which are fixed in a holder (53) of a removable venose sac (54) in whose upper part there is an infusion outlet (65), a second outlet (57) and a first outlet (55) which is hydraulically connected with a third outlet (56) to a pressure sensor (7), a third outlet (58) of the venose sac (54) is inserted into a bubble detector (59), the outputs of the first level detector (51), of the second level detector (52) and of the bubble detector (59) are electrically connected to the third connector (52).

8.    Apparatus according to Claims 5, 6 and 7 characterised in that the hydroblock for haemodialysis is formed by the blood cassette (15), underpressure cassette (14), conductivity cassette (13) and hydraulic interconnections (66) so that the by-pass outlet (30) from the conductivity cassette (13) is hydraulically connected through a by-pass interconnection (34) to the by-pass inlet (40) of the underpressure cassette (14), the first dialysate outlet (31) of the conductivity cassette (13) is hydraulically connected by dialysate interconnection (35) to the dialysate inlet (38) of the underpressure cassette (14) and the deaeration outlet (32) of the conductivity cassette (13) is hydraulically connected through a deaeration interconnection (36) containing the second jet (37) with deaeration inlet (42) of the underpressure cassette (14).

9.       Apparatus according to Claims 4 and 5 or Claim 8, characterised in that the blood cassette (15), underpressure cassette (14) and the conductivity cassette (13) are mutually interconnected by hydraulic interconnections (66) and together with low flow rate pumps (6) and pressure sensors (7) form a unit.

10.       Apparatus according to Claims 1, 2 and 8, characterised in that the monitor (2) contains three basic units (1) to which is connected the hydroblock (3) for haemodialysis, that into the socket (8) of the hydroblock (3) of the first basic unit (1) is inserted the first connector of the conductivity cassette (13), which has the first outlet (22) connected to the pressure sensor (7) of the first basic unit (1), into the socket (8) for the hydroblock (3) of the second basic unit (1) is inserted the second connector (49) of the underpressure cassette (14), which has the second outlet (44) connected to the pressure sensor (7) of the basic unit (1), and into the socket (8) for the hydroblock (3) of the third basic unit (1) is inserted the third connectord (62) of the blood cassette (15) whose third output (56) is connected to the pressure sensor (7) of the third basic unit (1) while the concentrate inlet (18) of the conductivity cassette (13) is hydraulically interconnected through the throttle valve (9) of the first basic unit (1) to the container with concentrate, the first inlet (24) and the first outlet (25) of the high flow rate pump (5) are connected to the high flow rate pump (5) of the first basic unit (1), to the second dialysate output (39) and to the third dialysate output (43) of the underpressure cassette (14) is connected the dialysation part (50) of the dialyser (64), the second inlet (47) of the high flow rate pump (5) is connected to the high flow rate pump (5) of the second basic unit (1) through whose by-pass throttle valve (9) there are led the by-pass interconnection (34) and the dialysate interconnection (35), the second outlet (48) of the high flow rate pump (5) is led to the waste drain for the dialysate, to the third outlet (58) of the venose sac (54) of the blood cassette (15) there is the venose inlet (60) connected being led through the throttling valve (9) of the third basic unit (1) led to the patient, the second outlet (57) of the venose sac (54) of the blood cassette (15) is led through the blood part (63) of the dialyser (64) and the high flow rate pump (5) of the third basic unit (1) to the arterial inlet (61).

11.     Apparatus according to Claims 1, 3 and 9 characterised in that the monitor (2) is composed of three basic units (1) upon which the hydroblock for haemodialysis (3) is slid so that into the socket (8) for hydroblock (3) of the first basic unit (1) is inserted the first connector (33), into the socket (8) for hydroblock (3) of the second basic unit (1) is inserted the second connector (49) and into the socket (8) for hydroblock (3) of the third basic unit (1) is inserted the third connector (62) while the inlet of the concentrate (18) of the conductivity cassette (13) is hydraulically interconnected through the throttle valve (9) of the first basic unit (1) to the container with the concentrate, the first inlet (24) and the first outlet (25) of the high flow rate pump (5) are interconnected to the high flow rate pump (5) of the first basic unit (1), to the second dialysate outlet (39) and the third dialysate outlet (43) of the underpressure cassette (14) is connected the dialysation part (50) of the dialyser (64), the second inlet (47) of the high flow rate pump (5) is connected to the high flow rate pump (5) of the second basic unit (1) through whose throttle valve (9) is led the by-pass interconnection (34) and the dialysate interconnection (35), the second outlet (48) of the high flow rate pump (5) is led into the waste outlet for the dialysate, the third outlet (58) of the venose sac (54) to the third output (58) of the venose sac (54) of the blood cassette (15) there is the venose inlet (60) connected leading through the throttling valve (9) of the third basic unit (1) to the patient.

FIG. 1

FIG. 2

FIG. 3

2/3

0165751

FIG.4